# EUROPÄISCHE PATENTSCHRIFT

(11) **EP 2 637 637 B1**
(45) Veröffentlichungstag und Bekanntmachung des Hinweises auf die Patenterteilung: **04.09.2019**
(21) Anmeldenummer: 11773438.4
(22) Anmeldetag: 12.10.2011
(51) Int. Cl.: A61K 8/42, A61Q 5/02, A61Q 5/12, A61Q 19/00, A61Q 19/10, A61K 8/37

(54) **ZUSAMMENSETZUNG ENTHALTEND MISCHUNGEN VON ISOSTEARINSÄUREAMID, GLYCERINESTER UND WASSER**
COMPOSITION COMPRISING MIXTURES OF ISOSTEARAMIDE, GLYCEROL ESTER AND WATER
COMPOSITION COMPRENANT DES MÉLANGES D'UN AMIDE D'ACIDE ISOSTÉARIQUE, D'ESTER DE GLYCÉROL ET D'EAU

(30) Priorität: 26.01.2011 DE 102011003170; 10.11.2010 DE 102010043675
(43) Veröffentlichungstag der Anmeldung: 18.09.2013
(73) Patentinhaber: Evonik Degussa GmbH, 45128 Essen (DE)
(72) Erfinder: HERRWERTH, Sascha, 45134 Essen (DE); SPRINGER, Oliver, 46485 Wesel (DE); WESTERHOLT, Ursula, 45138 Essen (DE)
(74) Vertreter: Evonik Patent Association
(86) Internationale Anmeldenummer: PCT/EP2011/067774
(87) Internationale Veröffentlichungsnummer: WO 2012/062519

(56) Entgegenhaltungen:
- EP-A1- 0 786 250
- EP-A1- 1 671 621
- WO-A1-02/080864
- WO-A1-02/092740
- WO-A2-2008/003090
- FR-A1- 2 691 472
- US-A- 5 198 209
- US-A1- 2003 012 759
- DATABASE GNPD [Online] Mintel; Juli 2010 (2010-07), "Shampoo Moisturiser", XP002667298, Database accession no. 1388145
- DATABASE GNPD [Online] Mintel; Juli 2010 (2010-07), "Active Shampoo Moisturiser", XP002667299, Database accession no. 1351644
- DATABASE GNPD [Online] Mintel; Juli 2010 (2010-07), "Hair Loss Schampoo", XP002667300, Database accession no. 1349975
- DATABASE GNPD [Online] Mintel; Februar 2010 (2010-02), "Neutral Liquid Soap", XP002667301, Database accession no. 1266952
- DATABASE GNPD [Online] Mintel; September 2009 (2009-09), "Aloe Vera Hydrating Shampoo", XP002667302, Database accession no. 1174615
- DATABASE GNPD [Online] Mintel; April 2009 (2009-04), "Anti-Dandruff Hair Loss Shampoo", XP002667303, Database accession no. 1078156
- DATABASE GNPD [Online] Mintel; Dezember 2008 (2008-12), "Hydrating Shampoo", XP002667304, Database accession no. 1021337
- DATABASE GNPD [Online] Mintel; November 2005 (2005-11), "Bath Ceremony Cream", XP002667305, Database accession no. 411907
- DATABASE GNPD [Online] Mintel; Februar 2005 (2005-02), "Shampoo", XP002667306, Database accession no. 10205935
- DATABASE GNPD [Online] Mintel; Juni 2002 (2002-06), "Gel de Bano Bath Gel", XP002667307, Database accession no. 153865
- DATABASE GNPD [Online] Mintel; März 2002 (2002-03), "Gel Leche Shower Gel", XP002667308, Database accession no. 142931
- DATABASE GNPD [Online] Mintel; Dezember 2002 (2002-12), "Body Wash Dermoprotector", XP002667309, Database accession no. 181470

## Beschreibung

Die vorliegende Erfindung ist gerichtet auf Zusammensetzungen, insbesondere Formulierungen zur Reinigung und Pflege von menschlichen oder tierischen Körperteilen, bestehend aus einer Mischung enthaltend mindestens ein Isostearinsäureamid, mindestens ein Glycerinester und Wasser gemäß Anspruch 1, sowie auf die Verwendung dieser Zusammensetzungen als Verdickungsmittel in reinigenden oder pflegenden Formulierungen, wie z.B. Shampoos, Konditionierer, Duschgele, Körperreinigungsmittel oder Hautreinigungsmittel oder zur Herstellung von Shampoos, Konditionierern, Duschgelen, Körperreinigungsmitteln oder Hautreinigungsmitteln.

Moderne kosmetische Reinigungsprodukte für Haut und/oder Haar, wie beispielsweise Duschbäder und Haarshampoos, weisen üblicherweise die folgenden Inhaltsstoffe auf:
- Wasser (als wichtigstem Lösemittel),
- Tenside,
- Viskositätsregulatoren / Verdicker zum Verdicken der Formulierung,
- Lösungsvermittler (Solubilisatoren) für wasserunlösliche Substanzen,
- Parfümöle,
- Konservierungsstoffe sowie
- Wirkstoffe zur Pflege von Haut und Haaren, wie z.B. Rückfetter.

Typische, nach dem Stand der Technik eingesetzte Verdicker oder Viskositätsregulatoren sind Kochsalz (NaCl), niedermolekulare nichtionische Tenside, wie Kokosfettsäuremonoethanolamid bzw. -diethanolamid (Cocamide MEA bzw. DEA) und Laureth-3, oder Polymere, hochmolekulare, assoziative, hochethoxylierte Fettderivate, wie z.B. PEG-200 Hydrogenated Glyceryl Palmate, PEG-150 Distearate und PEG-120 Methyl Glucose Dioleate.

Fettsäuremonoethanolamide und Fettsäurediethanolamide werden in einer Vielzahl von Anwendungen in der kosmetischen Industrie verwendet. Cocamide DEA (kommerziell erhältlich als REWOMID® DC 212 S, Evonik Goldschmidt GmbH) und Cocamide MEA (kommerziell erhältlich als REWOMID® C 212, Evonik Goldschmidt GmbH) sind in der Industrie Standardverdicker für wässrige tensidische Formulierungen, wie z. B. Handwaschseifen, Duschgele und Shampoos. Beide zeichnen sich durch eine sehr hohe verdickende Wirksamkeit aus.

Beide Produkte haben jedoch Nachteile:
Cocamide DEA ist eine potentielle Nitrosaminquelle, da ein sekundäres Amin als Rohstoff bei der Synthese verwendet wird.
Cocamide MEA ist keine potentielle Quelle unerwünschter Nitrosamine, liegt dafür aber bei Raumtemperatur als ein Feststoff vor und hat daher Nachteile bei der Verarbeitung, da ein Aufschmelzen des Rohstoffes und eine warme Verarbeitung der Formulierung notwendig sind.

Als Alternative ist bisher z.B. Isostearamide MIPA (Isostearinsäureamid des 1-Amino-2-propanols, kommerziell erhältlich als REWOMID® SPA, Evonik Goldschmidt GmbH) bekannt, welches bei Raumtemperatur flüssig ist und, da es auf einem primären Amin als Rohstoff basiert, keine potentielle Quelle unerwünschter Nitrosamine in kosmetischen Formulierungen darstellt. Nachteilig an diesem Rohstoff ist, dass dieser flüssige Rohstoff bei Abkühlung zum Separieren neigt und diese Separation nur durch Erwärmen auf 40 °C und Rühren rückgängig gemacht werden kann. Diese Separation ist besonders ausgeprägt, wenn das Produkt auf unter 15 °C abgekühlt wird, was beim Transport nur mit großem Aufwand ausgeschlossen werden kann. Analysen der separierenden Feststoffe haben ergeben, dass maßgeblich lineare Stearin- und Palmitinsäureamide des 1-Amino-2-propanols für die Separation verantwortlich sind. Diese linearen Fettsäuren sind in technischen Qualitäten von Isostearinsäure aber im Regelfall in geringen Mengen (ca. 2 bis 10 %) enthalten.

Ein Anwender muss deshalb entweder das gesamte Gebinde auf einmal verarbeiten oder das Produkt z. B. bei 40 °C durch Rühren homogenisieren. Damit stellt Isostearamide MIPA keine Alternative dar, die einen Vorteil gegenüber Cocamide MEA aufweist.

Aufgabe der vorliegenden Erfindung war deshalb die Bereitstellung eines alternativen Verdickers, der die Nachteile der aus dem Stand der Technik bekannten Verdicker nicht aufweist. Insbesondere sollte der Verdicker ohne Aufschmelzen und Homogenisierschritt bei Raumtemperatur verarbeitbar sein. Vorzugsweise sollte der Verdicker weiterhin bei Abkühlung (auf 5 °C) und Wiederaufheizen auf Raumtemperatur (22°C) keine Separation aufweisen und wie ursprünglich bei Raumtemperatur verarbeitbar sein.

Überraschenderweise wurde gefunden, dass diese Aufgabe durch eine Zusammensetzung gemäß Anspruch 1, die aus eine Mischung enthaltend Wasser, mindestens ein Isostearinsäureamid und mindestens einen Glycerinester besteht enthält, gelöst wird. So wurde insbesondere gefunden, dass Mischungen von Isostearamide MIPA mit Glycerinestern und Wasser, bevorzugt Mischungen aus Isostearamide MIPA, Glycerinlaurat und/oder Glycerincaprylat/caprat und Wasser, keine Separationstendenzen aufweisen und somit nicht die Schwächen des Isostearamide MIPA allein aufweisen.

Gegenstand der vorliegenden Erfindung sind deshalb Zusammensetzungen, insbesondere zur Reinigung und Pflege von menschlichen oder tierischen Körperteilen, bevorzugt Haut und Haar, bestehend aus einer Mischung enthaltend mindestens ein Isostearamid, mindestens ein Glycerinester und Wasser gemäß Anspruch 1. Ebenfalls Gegenstand der vorliegenden Erfindung ist die Verwendung dieser Zusammensetzungen als Verdickungsmittel in reinigenden oder pflegenden Formulierungen, wie z.B. Shampoos, Konditionierer, Duschgele, Körperreinigungsmittel oder Hautreinigungsmittel oder zur Herstellung von Shampoos, Konditionierern, Duschgelen, Körperreinigungsmitteln oder Hautreinigungsmitteln.

Die erfindungsgemäßen Zusammensetzungen gemäß Anspruch 1 enthaltend mindestens ein Isostearinsäureamid, mindestens ein Glycerinester und Wasser, vorzugsweise enthaltend ausschließlich mindestens ein Isostearinsäureamid, mindestens ein Glycerinester und Wasser haben den Vorteil, dass sie bei Raumtemperatur (22 °C) homogen und klar sind. Beim Abkühlen oder Einfrieren dieser Zusammensetzungen und anschließenden Erwärmen auf Raumtemperatur (22 °C) wird die Mischung wieder klar und ein aufwendiges Erwärmen auf 40 °C unter Rühren zur Homogenisierung kann vermieden werden.

Die erfindungsgemäßen Zusammensetzungen, bestehend aus Mischungen aus mindestens ein Isostearinsäureamid, mindestens ein Glycerinester und Wasser sowie weitere Komponenten weisen weiterhin den Vorteil auf, dass sie eine hohe Temperaturstabilität (15 - 40 °C) der Viskosität im Vergleich zu Formulierungen unter Verwendung marktüblicher Verdicker aufweisen. Dies ist daher von großer Bedeutung, da die Verwendung und Lagerung von z.B. Shampoos oder Duschgelen nicht immer bei Raumtemperatur erfolgt und ohne die Temperaturstabilität der Viskosität solche Formulierungen plötzlich wasserdünn sein können.

Ein weiterer Vorteil der erfindungsgemäßen Zusammensetzungen liegt darin, dass die Zusammensetzungen, insbesondere die darin enthaltenen Mischungen, sehr gute konditionierende Eigenschaften bei der Verwendung auf Haaren aufweisen. Es wurde überraschenderweise gefunden, dass die Mischung sowohl bereits alleine konditionierend wirkt, aber auch die Wirkung von Standardkonditionierern wie z.B. kationischen Polymeren oder Silikonderivate verstärkt.

Ein weiterer Vorteil der erfindungsgemäßen Zusammensetzungen liegt darin, dass die Zusammensetzungen, insbesondere die darin enthaltenen Mischungen, sehr gute, die Haut konditionierende Eigenschaften (das Hautgefühl verbessernde Eigenschaften) aufweist.

Die erfindungsgemäßen Zusammensetzungen, insbesondere die erfindungsgemäß vorhandenen Mischungen weisen außerdem den Vorteil auf, dass sie gute Schaumeigenschaften aufweisen. Zusammensetzungen, die neben der Mischung auch herkömmliche Tenside aufweisen führen zu deutlich besseren Schaumeigenschaften als sie solche nicht erfindungsgemäßen Zusammensetzungen aufweisen, die keine erfindungsgemäß vorhandene Mischung enthalten.

Die erfindungsgemäßen Zusammensetzungen, insbesondere die in diesen enthaltenden Mischungen, weisen außerdem den Vorteil auf, dass sie gute solubilisierende Eigenschaften besitzen. Diese Eigenschaft ermöglicht es in erfindungsgemäßen wässrigen tensidischen Zusammensetzungen kosmetische Öle und/oder Parfümöle klar (also ohne das Auftreten von Trübungen) zu lösen.

Noch ein weiterer Vorteil besteht darin, dass die Verwendung der erfindungsgemäßen Zusammensetzungen, insbesondere die in diesen enthaltenden Mischungen, auch in polyetherfreien tensidischen Formulierungen als Viskositätsregler, Pflegewirkstoff, Schaum-Booster oder Solubilisator möglich ist. Dies ist überraschend und ein besonderer Vorteil, da viele konventionelle Verdicker wie beispielsweise NaCl in polyetherfreien Formulierungen nicht wirksam sind, und hochmolekulare, assoziative Verdicker, die Polyethergruppen aufweisen, in polyetherfreien Formulierungen nicht eingesetzt werden können, da ansonsten die Formulierungen nicht mehr polyetherfrei wären.

Durch die hervorragenden Eigenschaften der in den erfindungsgemäßen Zusammensetzungen verwendeten Mischung(en) kann auf die Verwendung weiterer Verdicker/Viskositätsregulatoren und gegebenenfalls auch auf die Zugabe weiterer Rückfetter, Schaum-Booster oder Solubilisatoren in der jeweils gewählten Formulierung verzichtet werden.

Die erfindungsgemäßen Zusammensetzungen sowie deren Verwendungen werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere bezüglich der in Bezug genommenen Sachverhalte vollständig zum Offenbarungsgehalt der vorliegenden Erfindung gehören. Wenn im nachfolgenden Mittelwerte angegeben sind, so handelt es sich, wenn nicht anders angegeben, um zahlengemittelte Mittelwerte. Bei Prozentangaben handelt es sich, wenn nicht anders angegeben um Angaben in Gewichtsprozent.

Die erfindungsgemäßen Zusammensetzungen gemäß Anspruch 1 zeichnen sich dadurch aus, dass sie aus einer Mischung enthaltend mindestens ein Isostearinsäureamid, mindestens ein Glycerinester und Wasser bestehen.

Als Isostearinsäureamid können prinzipiell alle Isostearinsäureamide verwendet werden. Vorzugsweise enthält die Mischung als Isostearinsäureamid ein Amid, welches durch Umsetzung von Isostearinsäure oder Isostearinsäureestern wie z.B. Isostearinsäuremethylester mit 1-Amino-2-propanol, Monoethanolamin oder Diethanolamin, bevorzugt mit 1-Amino-2-propanol erhalten wird. Ein solches Amid wird üblicherweise als Isostearamide MIPA (INCI-Name) bezeichnet. Ein besonders geeignetes Isostearinsäureamid dieser Art ist beispielsweise bei der Evonik Goldschmidt GmbH unter der Bezeichnung REWOMID® SPA erhältlich.

Der Anteil an Isostearinsäureamid(en) an der Mischung beträgt von 50 bis 95 Gew.-%, bevorzugt von 70 bis 90 Gew.-% bezogen auf das Gesamtgewicht der Mischung.

Es kann vorteilhaft sein, wenn die erfindungsgemäße Mischung außerdem Amide aufweist, die durch Umsetzung von linearen Fettsäuren, vorzugsweise von linearen Fettsäuren mit 16 oder 18 Kohlenstoffatomen, bevorzugt von Palmitin- und/oder Stearinsäure mit 1-Amino-2-propanol, Monoethanolamin oder Diethanolamin, bevorzugt mit 1-Amino-2-propanol erhalten werden. Der Anteil dieser auf linearen Fettsäuren basierenden Amide in der Mischung beträgt vorzugsweise von 2 bis 10 Gew.-% bezogen auf die Summe aus Isostearinsäureamiden und Amiden von linearen Fettsäuren. Die linearen Amide sind häufig in kommerziell erhältlichen Isostearinsäureamiden enthalten. Der Anteil der auf linearen Fettsäuren basierenden Amide kann z. B. durch Gaschromatographie, z. B. mittels DGF-Methode DGF C-VI 10a bestimmt werden.

Als Glycerinester können prinzipiell alle Glycerinester eingesetzt werden. Die Glycerinester können Mischungen von Mono-, Di- und Triestern des Glycerins sein. Vorzugsweise werden Glycerinester eingesetzt, die eine Hydroxylzahl (OH-Zahl, bestimmt gemäß DIN 53240 (DGF-Methode C-V 2 17a)) von 200 bis 500 mgKOH/g, bevorzugt von 300 bis 475 aufweisen. Besonders bevorzugt werden hydrophile Glycerinester eingesetzt.

Als Säurekomponente weisen die Glycerinester solche Carbonsäuren auf, die von 8 bis 12 Kohlenstoffatome aufweisen. Ganz besonders bevorzugt enthält die Mischung als Glycerinester ein Glycerinlaurat, bevorzugt ein Glycerinmonolaurat, welches z.B. unter der Bezeichnung TEGIN® L 90 bei der Evonik Goldschmidt GmbH erhältlich ist, und/oder ein Glycerincaprylat/caprat, vorzugsweise ein Glycerinmonocaprylat/caprat, welches z.B. unter der Bezeichnung IMWITOR® 742 bei der SASOL Germany GmbH erhältlich ist.

Der Anteil an Glycerinester an der Mischung beträgt vorzugsweise von 1 bis 45 Gew.-%, bevorzugt von 5 bis 30 Gew.-% bezogen auf das Gesamtgewicht der Mischung.

Der Anteil an Wasser an der Mischung beträgt vorzugsweise von 0,1 bis 15 Gew.-%, bevorzugt von 2 bis 10 Gew.-% bezogen auf das Gesamtgewicht der Mischung.

Bevorzugte Zusammensetzungen weisen einen Anteil an Wasser von 1 bis 15 Gew.-%, bevorzugt von 2 bis 10 Gew.-%, einen Anteil von 1 bis 45 Gew.-%, bevorzugt von 5 bis 29 Gew.-%, einen Anteil an Isostearinsäureamid(en) von 50 bis 95 Gew.-%, bevorzugt von 70 bis 90 Gew.-% bezogen auf das Gesamtgewicht der Mischung auf. Besonders bevorzugte Zusammensetzungen weisen einen Anteil an auf linearen Fettsäuren basierenden Amide in der Mischung auf, der von 2 bis 10 Gew.-% bezogen auf die Summe aus Isostearinsäureamiden und Amiden von linearen Fettsäuren beträgt.

Eine solche nur die Mischung enthaltende Zusammensetzung kann z. B. als Verdickungsmittel verwendet werden. Die Mischung kann insbesondere zur Herstellung von Shampoo, Konditionierer, Duschgel, Körperreinigungsmittel oder Hautreinigungsmittel verwendet werden.

Zusammensetzungen ausgewählt aus Shampoo, Konditionierer, Duschgel, Körperreinigungsmittel und Hautreinigungsmittel, die neben der Mischung eine oder mehrere weitere Komponenten aufweisen, können vorzugsweise durch Mischen einer erfindungsgemäßen Zusammensetzung, die aus dieser Mischung besteht, mit einer weitere Komponente vorzugsweise durch Mischen mit Wasser und mindestens einer weiteren Komponente die von den in der Mischung vorhandenen Komponenten verschieden ist, erhalten werden. Das Mischen erfolgt vorzugsweise so, dass zumindest soviel Wasser und gegebenenfalls weitere der oben genannten Komponenten zugegeben werden, dass der Anteil des Wassers an der Gesamtzusammensetzung größer 35 Gew.-% beträgt

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

Alle Konzentrationen in den Anwendungsbeispielen sind, wenn nicht anders angegeben in Gewichtsprozent angegeben. Zur Herstellung der Zusammensetzungen wurden dem Fachmann bekannte, übliche Formulierungsverfahren eingesetzt.

### Beispiel 0: Herstellung von Mischungen von Isostearamid, Glycerinestern und Wasser

### Cap01 :

160 g REWOMID® SPA (Evonik Goldschmidt GmbH), 30 g TEGIN® L 90 (Evonik Goldschmidt GmbH) und 10 g destilliertes Wasser wurden in einem Dreihalskolben mit Thermometer und Rückflußkühler eingewogen und unter Rühren auf 60 °C erwärmt, bis eine homogen, klare Mischung erhalten wurde. Danach wurde das Produkt abgekühlt und es wurden 200 g einer hellgelben, klaren Flüssigkeit erhalten.

Diese erhaltene Mischung wurde in den folgenden Beispielen eingesetzt und wird im Folgenden mit Cap01 bezeichnet.

### Cap02:

166 g REWOMID® SPA (Evonik Goldschmidt GmbH), 30 g IMWITOR® 742 (SASOL Germany GmbH) und 4 g destilliertes Wasser wurden in einem Dreihalskolben mit Thermometer und Rückflußkühler eingewogen und unter Rühren auf 60 °C erwärmt, bis eine homogen, klare Mischung erhalten wurde. Danach wurde das Produkt abgekühlt und es wurden 200 g einer hellgelben, klaren Flüssigkeit erhalten.

Diese erhaltene Mischung wurde in den folgenden Beispielen eingesetzt und wird im Folgenden mit Cap02 bezeichnet.

### Cap03:

166 g REWOMID® SPA (Evonik Goldschmidt GmbH), 30 g Glycerinmonocaprylat/caprat, welches durch Reaktion von 1 mol Glycerin und 1 mol Capryl-/Caprinsäure (PRIFRAC 2912 der Firma Croda) selbst hergestellt wurde, und 4 g destilliertes Wasser wurden in einem Dreihalskolben mit Thermometer und Rückflußkühler eingewogen und unter Rühren auf 60 °C erwärmt, bis eine homogen, klare Mischung erhalten wurde. Danach wurde das Produkt abgekühlt und es wurden 200 g einer hellgelben, klaren Flüssigkeit erhalten.

Diese erhaltene Mischung wurde in den folgenden Beispielen eingesetzt und wird im Folgenden mit Cap03 bezeichnet.

### Beispiel 1: Austestung der Verdickungseigenschaften

Die verdickende Wirkung des Cap01 aus Beispiel 0 wurde im Vergleich zu gängigen tensidischen Verdickern in verschiedenen Tensidsystemen getestet. Die Viskosität wurden mittels eines Brookfield Viskosimeters (Brookfield LVF, Spindel 3, 5 Upm) bei 25 °C gemessen.

### Beispiel 1.1: Tensidsystem 1a:

32 Gew.-% Natriumlaurylethersulfat (Cognis, Texapon® NSO, 28 %-ig) und 9 Gew.-% Sodium Cocoamphoacetate (Evonik Goldschmidt GmbH, Rewoteric® AM C, 32 %-ig) wurde auf eine Viskosität von 3500 mPas bei 25 °C eingestellt. Die dazu jeweils benötigte Verdickerkonzentration ist in Tabelle 1-1 dargestellt. Es zeigt sich, dass Cap01 (Beispiel 1.1a) im Vergleich zu marktüblichen, nicht erfindungsgemäßen Verdickern (Beispiele 1.1b und 1.1c) am effektivsten ist, da die geringste Einsatzkonzentration benötigt wird.

**Tabelle 1-1: Verdickungswirkung von Cap01 im Vergleich zu marktüblichen Verdickern (Angaben in Gew.-%)**

| Beispiel | 1.1a | 1.1b | 1.1c |
|---|---|---|---|
| Texapon® NSO (Cognis, INCI: Sodium Laureth Sulfate, 28 %-ig) | 32,0 | 32,0 | 32,0 |
| Rewoteric® AM C (Evonik Goldschmidt GmbH, INCI: Sodium Cocoamphoacetate, 32 %-ig) | 9,0 | 9,0 | 9,0 |
| Cap01 | 1,2 | | |
| REWOMID® DC 212 S (Evonik Goldschmidt GmbH, INCI: Cocamide DEA)* | | 3,6 | |
| REWOMID® C 212, (Evonik Goldschmidt GmbH, INCI: Cocamide MEA)* | | | 2,0 |
| Wasser, demineralisiert | ad 100,0 | | |
| Viskosität [mPas], | 3500 | | |

### Beispiel 1.2: Tensidsystem 1b:

17,9 Gew.-% Natriumlaurylethersulfat (Cognis, Texapon® NSO, 28 %-ig), 6,6 Gew.-% Cocamidopropylbetain (Evonik Goldschmidt GmbH, TEGO® Betain F 50, 38 %-ig) und 6,3 Gew.-% Disodium Laureth Sulfosuccinate (Evonik Goldschmidt GmbH, Rewopol® SB FA 30 B, 40 %-ig) wurde auf eine Viskosität von 3500 mPas bei 25 °C eingestellt. Bei dieser Zusammensetzung handelt es sich um eine milde, schwer zu verdickende Tensidformulierung.

In Tab. 1-2 wird dargestellt, welche Einsatzkonzentration des marktüblichen, nicht erfindungsgemäßen Verdickers (Beispiel 1.2b) REWOMID® DC 212 S (Evonik Goldschmidt GmbH, INCI: Cocamide DEA) im Vergleich zu der Mischung Cap01 (Beispiel 1.2a) benötigt wurde. Es wird offensichtlich, dass die Mischung Cap01 eine deutlich höhere Effektivität aufweist. Ferner wurde auch der marktübliche Verdicker REWOMID® C 212 (Evonik Goldschmidt GmbH, INCI: Cocamide MEA) in diesem Tensidsystem getestet, wobei keine ausreichende Verdickung und Trübungen beobachtet wurden. Cocamide MEA kann in diesem Tensidsystem daher nicht als Verdicker verwendet werden.

**Tabelle 1-2: Verdickungswirkung von Cap01 in einer milden, schwer zu verdickenden Formulierung im Vergleich zu marktüblichen Verdickern (Angaben in Gew.-%).**

| Beispiel | 1.2a | 1.2b |
|---|---|---|
| Texapon® NSO (Cognis, INCI: Sodium Laureth Sulfate, 28 %-ig) | 17,9 | 17,9 |
| Rewopol® SB FA 30 B(Evonik Goldschmidt GmbH, INCI: Disodium Laureth Sulfosuccinate, 40 %-ig) | 6,3 | 6,3 |
| TEGO® Betain F 50 (Evonik Goldschmidt GmbH, INCI: Cocamidopropyl Betaine, 38 %-ig) | 6,6 | 6,6 |
| NaCl | 1 | 1 |
| Cap01 | 1,7 | |
| REWOMID® DC 212 S (Evonik Goldschmidt GmbH, INCI: Cocamide DEA) | | 3,8 |
| Wasser, demineralisiert | ad 100,0 | |
| Viskosität [mPas], | 3500 | |

### Beispiel 1.3: Temperaturabhängigkeit der Viskosität in Tensidsystem 1c

32 Gew.-% Natriumlaurylethersulfat (Cognis, Texapon® NSO, 28%-ig), 8 Gew.-% Cocamidopropylbetain (Evonik Goldschmidt GmbH, TEGO® Betain F 50, 38%-ig) und 0,7 Gew.-% NaCl, wurde auf eine Viskosität von 4200 mPas bei 25 °C eingestellt. Die dazu jeweils benötigte Verdickerkonzentration ist in Tabelle 1-3 dargestellt, wobei in Beispiel 1.3a erfindungsgemäßer Verdicker und in den Beispielen 1.3b und 1.3c nicht erfindungsgemäße handelsübliche Verdicker eingesetzt wurden. Anschließend wurden die Zusammensetzungen für 12 Stunden auf 40 °C erwärmt und die Viskositäten der Zusammensetzungen erneut gemessen. Es wurde beobachtet, dass die Zusammensetzung bei der Verwendung von Cap01 die höchste Temperaturstabilität aufweist.

**Tabelle 1-3: Temperaturstabilität der Viskosität von Zusammensetzungen unter Verwendung von Cap01 im Vergleich zu marktüblichen Verdickern (Angaben in Gew.-%).**

| Beispiel | 1.3a | 1.3b | 1.3c |
|---|---|---|---|
| Texapon NSO® (Cognis, INCI: Sodium Laureth Sulfate, 28 %-ig) | 32,0 | 32,0 | 32,0 |
| TEGO® Betain F 50 (Evonik Goldschmidt GmbH, INCI: Cocamidopropyl Betaine, 38 %-ig) | 8,0 | 8,0 | 8,0 |
| Cap01 | 1,0 | | |
| REWOMID® DC 212 S (Evonik Goldschmidt GmbH, INCI: Cocamide DEA) | | 1,6 | |
| REWOMID® C 212 (Evonik Goldschmidt GmbH, INCI: Cocamide MEA) | | | 1,0 |
| NaCl | 0,7 | 0,7 | 0,7 |
| Wasser, demineralisiert | ad 100,0 | | |
| Viskosität [mPas] bei 25 °C | 4200 | 4200 | 4200 |
| Viskosität [mPas] bei 40 °C | 3100 | 500 | 200 |

### Beispiel 1.4: Temperaturabhängigkeit der Viskosität in Tensidsystem 1b

Die Formulierungen mit Tensidsystem 1b (siehe Tabelle 1-2) wurden ebenfalls auf 40 °C über Nacht erwärmt und erneut die Viskositäten bestimmt (Tabelle 1-4). Es wurde beobachtet, dass die Zusammensetzung bei der Verwendung von Cap01 die höchste Temperaturstabilität aufweist.

**Tabelle 1-4: Temperaturstabilität der Viskosität von Zusammensetzungen unter Verwendung von Cap01 im Vergleich zu REWOMID® DC 212 S (Evonik Goldschmidt GmbH, INCI: Cocamide DEA). Angaben in Gew.-%.**

| Beispiel | 1.4a | 1.4b |
|---|---|---|
| Texapon® NSO (Cognis, INCI: Sodium Laureth Sulfate, 28 %-ig) | 17,9 | 17,9 |
| Rewopol® SB FA 30 B(Evonik Goldschmidt GmbH, INCI: Disodium Laureth Sulfosuccinate, 40 %-ig) | 6,3 | 6,3 |
| TEGO® Betain F 50 (Evonik Goldschmidt GmbH, INCI: Cocamidopropyl Betaine, 38 %-ig) | 6,6 | 6,6 |
| NaCl | 1 | 1 |
| Cap01 | 1,7 | |
| REWOMID® DC 212 S (Evonik Goldschmidt GmbH, INCI: Cocamide DEA) | | 3,8 |
| Wasser, demineralisiert | ad 100,0 | |
| Viskosität [mPas] bei 25 °C | 3500 | 3500 |
| Viskosität [mPas] bei 40 °C | 2800 | 450 |

### Beispiel 2: Austestung der Konditionierung von Haut (Hautpflegeleistung) und Schaumeigenschaften mittels eines Handwaschtests

Zur Bewertung der rückfettenden Pflege von Haut (Hautpflegeleistung) und der Schaumeigenschaften von Cap01 in wässrigen, tensidischen Zusammensetzungen (Tensidformulierungen) wurden sensorische Handwaschtests im Vergleich zu dem Marktstandard Polyethylenglykol(7)glycerylmonoacetat durchgeführt. Polyethylenglykol(7)glycerylmonococoat ist in der Industrie als rückfettender Pflegewirkstoff weit verbreitet und gilt als hochwirksame Komponente in wässrigen, tensidischen Formulierungen.

Eine Gruppe bestehend aus 10 trainierten Prüfpersonen wusch sich dabei definiert die Hände und bewertete Schaumeingeschaften und Hautgefühl anhand einer Notenskala von 1 (schlecht) bis 5 (sehr gut). Die eingesetzten Produkte wurden jeweils in einer standardisierten Tensidformulierung (Tabelle 2-1) getestet.

Als Kontrollformulierung 2a wird eine Tensidformulierung ohne Zusatz eines Additivs verwendet. Die Tensidformulierung 2b stellt die erfindungsgemäße Zusammensetzung und die Tensidformulierung 2c eine nicht erfindungsgemäße Zusammensetzung dar (Tabelle 2-1).

**Tabelle 2-1: Testformulierungen für Handwaschtest gemäß Beispiel 2.1 (Angaben in Gew.-%).**

| Formulierungs-Beispiele | 2a | 2b | 2c |
|---|---|---|---|
| Texapon NSO® (Cognis, INCI: Sodium Laureth Sulfate, 28 %-ig) | 32 | 32 | 32 |
| TEGO® Betain F 50 (Evonik Goldschmidt GmbH INCI: Cocamidopropyl Betaine, 38 %-ig) | 8 | 8 | 8 |
| NaCl | 1,5 | 1,5 | 1,5 |
| Wasser, demineralisiert | ad. 100 | | |
| Cap01 | | 1.0 | |
| Tegosoft® GC (Evonik Goldschmidt GmbH, INCI: Polyethylenglykol(7)glycerylmonococoat) | | | 1.0 |

In Tabelle 2-2 sind die Ergebnisse des Handwaschtests dargestellt. Anhand der Messergebnisse wird ersichtlich, dass die erfindungsgemäße Zusammensetzung 2b unter Verwendung von Cap01 eine bessere Hautglätte und Hautweichheit 3 Minuten nach der Applikation und ein überlegenes Hautgefühl während des Waschens im Vergleich zu den Vergleichszusammensetzungen 2a und 2c nach dem Stand der Technik bewirken. Des Weiteren ist anhand der Messwerte ersichtlich, dass die erfindungsgemäße Zusammensetzung 2b mit Cap01 eine Verbesserung der Schaumeigenschaften zu den Zusammensetzungen nach dem Stand der Technik bewirkt.

**Tabelle 2-2: Ergebnisse des Handwaschtests gemäß Beispiel 2.1**

| Testformulierung | 2a | 2b | 2c |
|---|---|---|---|
| Anschäumverhalten | 2,65 | 3,95 | 3,0 |
| Schaumvolumen | 2,75 | 3,3 | 3,1 |
| Schaumcremigkeit | 2,75 | 3,25 | 3,15 |
| Hautglätte nach 3 min. | 2,95 | 3,95 | 3,65 |
| Hautweichheit nach 3 min. | 3,1 | 4,1 | 3,35 |

### Beispiel 3: Austestung der solubilisierenden Eigenschaften

Die solubilisierenden Eigenschaften von Cap01 wurden getestet, indem das wasserunlösliches Öl Isopropylmyristat (Evonik Goldschmidt GmbH, TEGOSOFT® M) in einer Tensidlösung, bestehend aus 40 Gew.-% Natriumlaurylethersulfat (Cognis, Texapon® NSO, 28 %-ig), 10 Gew.-% Cocamidopropylbetain (Evonik Goldschmidt GmbH, TEGO® Betain F 50, 38%-ig) und 0,5 Gew.-% Solubilisator-Zusatz (siehe Beispiele 3.3, 3.4, 3.6 und 3.7) klar gelöst wurde. Zum Vergleich wurde das Öl in der reinen Tensidlösung (siehe Beispiele 3.1 und 3.2 in Tabelle 3-1) ohne Solubilisator-Zusatz gelöst. Als Marktstandard wurde PEG-7 Glyceryl Cocoate (Evonik Goldschmidt GmbH, TEGOSOFT® GC) (siehe Beispiele 3.6, 3.7 und 3.8 in Tabelle 3-1) eingesetzt.

Tabelle 3-1 gibt die Menge Isopropylmyristat (Evonik Goldschmidt GmbH, TEGOSOFT® M) an, die im jeweiligen System noch klar gelöst werden konnte. Oberhalb dieser Menge kommt es zu Trübungen.

**Tab. 3-1: Formulierungen und Ergebnisse - Solubilisierungsversuche (Angaben in Gew.-%).**

| Beispiel | 3.1 | 3.2 | 3.3 | 3.4 | 3.5 | 3.6 | 3.7 | 3.8 |
|---|---|---|---|---|---|---|---|---|
| Texapon® NSO (Cognis, INCI: Sodium Laureth Sulfate, 28%-ig) | 40 | 40 | 40 | 40 | 40 | 40 | 40 | 40 |
| TEGO® Betain F 50 (Evonik Goldschmidt GmbH, INCI: Cocamidopropyl Betaine, 38%-ig) | 10 | 10 | 10 | 10 | 10 | 10 | 10 | 10 |
| TEGOSOFT® M (Evonik Goldschmidt GmbH, INCI: Isopropyl Myristate) | 0,5 | 0,6 | 1,0 | 1,7 | 1,8 | 1,0 | 1,1 | 1,2 |
| Cap01 | | | 0,5 | 0,5 | 0,5 | | | |
| TEGOSOFT® GC (Evonik Goldschmidt GmbH, INCI: PEG-7 Glyceryl Cocoate) | | | | | | 0,5 | 0,5 | 0,5 |
| Wasser | ad 100,0 | | | | | | | |
| Aussehen | klar | trüb | klar | klar | trüb | klar | klar | trüb |

Den in Tabelle 3-1 angegebenen Ergebnissen kann entnommen werden, dass Cap01 eine deutliche solubilisierende Wirkung zeigt, die den Marktstandard PEG-7 Glyceryl Cocoate (Evonik Goldschmidt GmbH, TEGOSOFT® GC) übertrifft.

### Beispiel 4: Austestung der Konditionierung von Haar mittels Sensoriktests:

Für die anwendungstechnische Beurteilung der Konditionierung von Haar wurden die erfindungsgemäße Mischung Cap01 in einfachen Shampoo Formulierungen eingesetzt. Die Anwendungseigenschaften beim Einsatz in einem Shampoo wurden in den in Tabelle 4-1 angegebenen Rezepturen überprüft. Die Beispiele 4.1 und 4.3 stellen Vergleichsbeispiele dar.

**Tabelle 4-1: Shampooformulierungen zur Austestung der Haar-konditionierenden Eigenschaften von der Mischung Cap01 (Angaben in Gew.-%).**

| Formulierungs-Beispiele | 4.1 | 4.2 | 4.3 | 4.4 |
|---|---|---|---|---|
| Texapon NSO®, 28 %-ig, Cognis (INCI: Sodium Laureth Sulfate) | 32 | 32 | 32 | 32 |
| TEGO® Betain F 50, 38 %-ig, Evonik Goldschmidt GmbH (INCI: Cocamidopropyl Betaine) | 8 | 8 | 8 | 8 |
| Jaguar 162, Rhodia (INCI: Guar Hydroxypropyl trimonium Chloride; Kationisches Polymer zur Verbesserung der Wirksamkeit von Konditioniermitteln) | | | 0,3 | 0,3 |
| Wasser, demineralisiert | Ad 100,0 | | | |
| Zitronensäure | Ad. pH 6,0 | | | |
| Mischung Cap01 | | 0,5 | | 0,5 |

Die Vorbehandlung der Haare erfolgt im Falle der Eigenschaftsprüfung von Haarspülungen durch ein Shampoo, welches keine Konditioniermittel enthält.

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet. Der Testablauf, die verwendeten Basismaterialien sowie die Details der Beurteilungskriterien sind in DE 103 27 871 beschrieben.

### Standardisierte Behandlung von vorgeschädigten Haarsträhnen mit konditionierenden Proben

Die, wie oben beschrieben, vorgeschädigten Haarsträhnchen wurden wie folgt mit dem oben beschriebenen Shampoo behandelt: Die Haarsträhnen wurden unter fließendem, warmem Wasser benetzt. Das überschüssige Wasser wurde leicht von Hand ausgedrückt, dann wurd das Shampoo aufgebracht und sanft im Haar eingearbeitet (1 ml/Haarsträhne (2 g)). Nach einer Verweilzeit von 1 min wurde das Haar für 1 min gespült.

### Beurteilungskriterien:

Die sensorischen Bewertungen erfolgten nach Noten, die auf einer Skala von 1 bis 5 vergeben werden, wobei 1 die schlechteste und 5 die beste Bewertung ist. Die einzelnen Testkriterien erhielten jeweils eine eigene Bewertung. Die Testkriterien waren: Nasskämmbarkeit, Nassgriff.

In der folgenden Tabelle 4-2 werden die Ergebnisse der sensorischen Beurteilung der wie oben beschrieben durchgeführten Behandlung der Haarstränchen mit den erfindungsgemäßen Formulierungen 4.2 und 4.4, und der Vergleichsformulierungen 4.1 und 4.3 verglichen.

**Tabelle 4-2: Ergebnisse der Konditionierung von Haar aus Shampoo-Formulierung**

| | Nasskämmbarkeit | Nassgriff |
|---|---|---|
| Vergleichsformulierung 4a1 | 2,0 | 1,9 |
| Erfindungsgemäße Formulierung 4a2 | 3,0 | 3,1 |
| Vergleichsformulierung 4a3 | 3,1 | 3,1 |
| Erfindungsgemäße Formulierung 4a4 | 3,9 | 3,6 |

Die Ergebnisse zeigen in überraschender Weise, dass die erfindungsgemäßen Zusammensetzungen (Formulierungen 4.2 und 4.4) mit der Mischung Cap01 signifikant bessere Bewertungen erhalten, als die entsprechenden Vergleichsformulierungen 4.1 und 4.3 ohne den Zusatz der Mischung Cap01. Es konnte somit gezeigt werden, dass die Mischung Cap01 sowohl in Formulierungen die keine weiteren konditionierenden Verbindungen enthalten (Vergleich 4.1 zu 4.2) als auch in Formulierungen die Guarquat als Basiskonditionierer enthalten (Vergleich 4.3 zu 4.4) einen konditionierenden Effekt haben.

Die angegebenen Beispiele belegen somit die pflegende, schaumfördernde, konditionierende und/oder solubilisierende Wirkung der erfindungsgemäßen Zusammensetzung Cap01. Ferner konnte die hohe Wirksamkeit der Zusammensetzung Cap01 als Verdicker in verschiedenen Tensidsystemen gezeigt werden, wobei die Wirksamkeit der Vergleichssubstanzen (Marktstandards) teilweise deutlich übertroffen wurde. Des Weiteren konnten die hohe Viskositäts-Temperaturstabilität der Zusammensetzungen mit der Mischung Cap01 als Verdicker in verschiedenen Tensidsystemen gezeigt werden, wobei die Wirksamkeit der Vergleichssubstanzen nach dem Stand der Technik deutlich übertroffen wird.

### Beispiel 5: Weitere Formulierungsbeispiele:

Die in den nachfolgenden Tabellen 5-1 bis 5-28 angegebenen Formulierungsbeispiele 1 bis 28 zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet.

Falls eine zweiphasige Herstellung erforderlich ist, werden die beiden Phasen mit A und B in den angegebenen Tabellen gekennzeichnet. Bei dreiphasigen Prozessen werden die drei Phasen mit A, B und C benannt. Wenn nicht anders angegeben handelt es sich bei den Angaben in den Tabellen 5-1 bis 5-28 um Angaben in Gew.-%.

**Tabelle 5-1: Formulierungsbeispiel 1, Shampoo, PEG- & Sulfat frei**

| | |
|---|---|
| REWOTERIC® AMC, Evonik Goldschmidt GmbH, 32%-ig, (INCI: Sodium Cocoamphoacetate) | 15,00 |
| REWOPOL® SB F 12 P, Evonik Goldschmidt GmbH, 96%-ig, (INCI: Disodium Lauryl Sulfosuccinate) | 3,80 |
| Cap01 | 0,50 |
| Perfume | 0,30 |
| Water | 66,10 |
| TEGO® Betain F 50, Evonik Goldschmidt GmbH, 38%-ig, (INCI: Cocamidopropyl Betaine) | 13,00 |
| ANTIL® HS 60, Evonik Goldschmidt GmbH, (INCI: Cocamidopropyl Betaine; Glyceryl Laurate) | 1,00 |
| Citric Acid, 30-ig% | q.s. |
| Preservative | 0,30 |

**Tabelle 5-2: Formulierungsbeispiel 2, mildes Haar- und Körperreinigungsmittel**

| | |
|---|---|
| Plantacare® 1200 UP, Cognis, 50%-ig, (INCI: Lauryl Glucoside) | 11,40 |
| Plantacare® 818 UP, Cognis, 51%-ig, (INCI: Coco Glucoside) | 5,60 |
| Water | 63,00 |
| Cap02 | 0,50 |
| TEGOSOFT® LSE 65 K SOFT, Evonik Goldschmidt GmbH, (INCI: Sucrose Cocoate) | 1,50 |
| TEGO® Betain F 50, Evonik Goldschmidt GmbH, 38%-ig, (INCI: Cocamidopropyl Betaine) | 18,00 |
| Perfume,preservative | q.s. |
| Citric Acid, 30% | q.s. |

**Tabelle 5-3: Formulierungsbeispiel 3, feuchtigkeitspendendes Hautreinigungsmittel**

| | | |
|---|---|---|
| A | TEXAPON® NSO, Cognis, 28%-ig, (INCI: Sodium Laureth Sulfate) | 30,00 |
| | Cap03 | 0,70 |
| | Perfume | 0,30 |
| B | Water | 55,40 |
| | TEGOCEL® fluid HPM 4000, Evonik Goldschmidt GmbH, (INCI: Hydroxypropyl Methylcellulose) | 1,20 |
| | TEGO® Betain C 60, Evonik Goldschmidt GmbH, 46%-ig, (INCI: Cocamidopropyl Betaine) | 8,10 |
| | TEGOSOFT® APM, Evonik Goldschmidt GmbH, (INCI: PPG-3 Myristyl Ether) | 1,00 |
| | TEGO® Pearl N 300, Evonik Goldschmidt GmbH, (INCI: Glycol Distearate; Laureth-4; Cocamidopropyl Betaine) | 2,00 |
| | REWODERM® LI S 80, Evonik Goldschmidt GmbH, (INCI: PEG-200 Hydrogenated Glyceryl Palmate; PEG-7 Glyceryl Cocoate) | 1,00 |
| | Preservative | 0,60 |
| | Citric Acid, 30%-ig | q.s. |

**Tabelle 5-4: Formulierungsbeispiel 4, klares Duschgel**

| | |
|---|---|
| Cap01 | 1,00 |
| TAGAT® CH 40, Evonik Goldschmidt GmbH, (INCI: PEG-40 Hydrogenated Castor Oil) | 2,50 |
| Perfume | 0,30 |
| TEXAPON® NSO, Cognis, 28%-ig, (INCI: Sodium Laureth Sulfate) | 42,90 |
| Water | 39,30 |
| TEGO® Betain F 50, Evonik Goldschmidt GmbH, 38%-ig, (INCI: Cocamidopropyl Betaine) | 10,70 |
| LACTIL®, Evonik Goldschmidt GmbH, (INCI: Sodium Lactate; Sodium PCA; Glycine; Fructose; Urea; Niacinamide; Inositol; Sodiumbenzoate; Lactic Acid) | 1,00 |
| ANTIL® 171, Evonik Goldschmidt GmbH, (INCI: PEG-18 Glyceryl Oleate/Cocoate) | 2,00 |
| Preservative | 0,30 |

**Tabelle 5-5: Formulierungsbeispiel 5, Clear Shower Gel**

| | |
|---|---|
| TEXAPON® NSO, Cognis, 28%-ig, (INCI: Sodium Laureth Sulfate) | 37,00 |
| Cap02 | 1,00 |
| Perfume | 0,30 |
| Water | 42,00 |
| REWOTERIC® AMC, Evonik Goldschmidt GmbH, 32%-ig, (INCI: Sodium Cocoamphoacetate) | 9,00 |
| TEGO® Betain 810, Evonik Goldschmidt GmbH, 38%-ig, (INCI: Capryl/Capramidopropyl Betaine) | 7,60 |
| LACTIL®, Evonik Goldschmidt GmbH, (INCI: Sodium Lactate; Sodium PCA; Glycine; Fructose; Urea; Niacinamide; Inositol; Sodiumbenzoate; Lactic Acid) | 1,00 |
| Citric Acid, 30%-ig | 1,30 |
| REWODERM® LI S 80, Evonik Goldschmidt GmbH, (INCI: PEG-200 Hydrogenated Glyceryl Palmate; PEG-7 Glyceryl Cocoate) | 0,50 |
| Preservative | 0,30 |

**Tabelle 5-6: Formulierungsbeispiel 6, Shampoo, PEG- & sulfate free**

| | |
|---|---|
| REWOTERIC® AM C, Evonik Goldschmidt GmbH, 32%-ig, (INCI: Sodium Cocoamphoacetate) | 15,00 |
| Plantapon ACG 50, Cognis (INCI: Disodium Cocoyl Glutamate) | 3,80 |
| Cap01 | 1,00 |
| Perfume | 0,30 |
| Water | 66,30 |
| TEGO® Betain F 50, Evonik Goldschmidt GmbH, 38%-ig, (INCI: Cocamidopropyl Betaine) | 10,00 |
| VARISOFT® PATC, Evonik Goldschmidt GmbH, (INCI: Palmitamidopropyltrimonium Chloride) | 2,30 |
| REWOMID® SPA, Evonik Goldschmidt GmbH, (INCI: Isostearamide MIPA) | 1,00 |
| Preservative | 0,30 |
| Citric Acid, 30 %-ig | q.s. |

**Tabelle 5-7: Formulierungsbeispiel 7, Duschgel**

| | |
|---|---|
| TEXAPON® NSO, Cognis, 28%-ig (INCI: Sodium Laureth Sulfate) | 15,00 |
| Cap03 | 0,50 |
| Perfume | 0,30 |
| PGFAC-S, Cognis (INCI: Sodium cocoyl hydrolyzed wheat protein glutamate) | 1,50 |
| REWOPOL SB CS 50 B, Evonik Goldschmidt GmbH, 40%-ig, (INCI: Disodium PEG-5 Laurylcitrate Sulfosuccinate; Sodium Laureth Sulfate) | 7,50 |
| Water | 60,10 |
| TEGO® Betain F 50, Evonik Goldschmidt GmbH, 38%-ig, (INCI: Cocamidopropyl Betaine) | 9,00 |
| TEGO® Betain 810, Evonik Goldschmidt GmbH, 38%-ig, (INCI: Capryl/Capramidopropyl Betaine) | 4,00 |
| ANTIL® 200, Evonik Goldschmidt GmbH, (INCI: PEG-200 Hydrogenated Glyceryl Palmate; PEG-7 Glyceryl Cocoate) | 1,80 |
| Preservative | 0,30 |

**Tabelle 5-8: Formulierungsbeispiel 8, Shampoo, PEG- & sulfate free**

| | | |
|---|---|---|
| A | REWOTERIC® AMC, Evonik Goldschmidt GmbH, 32%-ig, (INCI: Sodium Cocoamphoacetate) | 20,00 |
| | REWOPOL® SB F 12 P, Evonik Goldschmidt, 96%-ig, (INCI: Disodium Lauryl Sulfosuccinate) | 5,90 |
| | Cap01 | 0,70 |
| B | Water | 66,20 |
| | Citric Acid, 30%-ig | 3,60 |
| C | ANTIL® HS 60, Evonik Goldschmidt GmbH, (INCI: Cocamidopropyl Betaine; Glyceryl Laurate) | 3,00 |
| | Preservative | 0,60 |

**Tabelle 5-9: Formulierungsbeispiel 9, Körperreinigungsmittel**

| | | |
|---|---|---|
| A | TEXAPON® NSO Cognis 28%-ig,(INCI: Sodium Laureth Sulfate | 30,00 |
| | Cap01 | 0,50 |
| | ABIL® B 8832, Evonik Goldschmidt GmbH, (INCI: Bis-PEG/PPG-20/20 Dimethicone) | 0,30 |
| | Perfume | 0,30 |
| B | Water | 53,00 |
| | TEGOCEL® fluid HPM 4000, Evonik Goldschmidt GmbH, (INCI: Hydroxypropyl Methylcellulose) | 1,20 |
| | Citric Acid Monohydrate | 0,50 |
| | REWOTERIC® AM C, Evonik Goldschmidt GmbH, 32%-ig, (INCI: Sodium Cocoamphoacetate) | 10,00 |
| | TEGO® Pearl N 300, Evonik Goldschmidt GmbH, (INCI: Glycol Distearate; Laureth-4; Cocamidopropyl Betaine) | 2,00 |
| | REWODERM® LI S 80, Evonik Goldschmidt GmbH, (INCI: PEG-200 Hydrogenated Glyceryl Palmate; PEG-7 Glyceryl Cocoate) | 1,60 |
| | Preservative | 0,60 |
| | Citric Acid, 30%-ig | q.s. |

**Tabelle 5-10: Formulierungsbeispiel 10, Sprühbare Haarmilch, PEG-free**

| | | |
|---|---|---|
| A | Water | 95,30 |
| | Lactic Acid, 80%-ig | 0,40 |
| B | TEGO® AMID S 18, Evonik Goldschmidt GmbH, (INCI: Stearamidopropyl Dimethylamine) | 1,20 |
| | TEGIN® G 1100 Pellets, Evonik Goldschmidt GmbH, (INCI: Glycol Distearate) | 0,60 |
| | TEGO® Care PS, Evonik Goldschmidt GmbH, (INCI: Methyl Glucose Sesquistearate) | 1,20 |
| | TEGOSOFT® DEC, Evonik Goldschmidt GmbH, (INCI: Diethylhexyl Carbonate) | 0,30 |
| | Cap01 | 1,00 |
| | Perfume, preservative | q.s. |

**Tabelle 5-11: Formulierungsbeispiel 11, Body cleansing Foam**

| | |
|---|---|
| TEXAPON® NSO, Cognis, 28%-ig (INCI: Sodium Laureth Sulfate) | 14 |
| Perfume | 0,3 |
| Cap01 | 0,2 |
| REWOTERIC® AM C, Evonik Goldschmidt GmbH, 32%-ig (INCI: Sodium Cocoamphoacetate) | 8 |
| Water | 75,5 |
| TEGOCEL® HPM 50, Evonik Goldschmidt GmbH (INCI: Hydroxypropyl Methylcellulose) | 0,5 |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1 |
| Citric Acid Monohydrate | 0,5 |

**Tabelle 5-12: Formulierungsbeispiel 12, Clear Conditioning Shampoo**

| | |
|---|---|
| TEXAPON® NSO, Cognis, 28%-ig (INCI: Sodium Laureth Sulfate) | 32,00 |
| VARISOFT® PATC, Evonik Goldschmidt GmbH(INCI: Palmitamidopropyltrimonium Chloride) | 1,50 |
| REWODERM® LI S 80, Evonik Goldschmidt GmbH (INCI: PEG-200 Hydrogenated Glyceryl Palmate; PEG-7 Glyceryl Cocoate) | 2,00 |
| Cap01 | 0,50 |
| Perfume | 0,25 |
| Water | 54,05 |
| TEGO® Cosmo C 100, Evonik Goldschmidt GmbH, (INCI: Creatine) | 1,00 |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00 |
| NaCl | 0,50 |
| Preservative | q.s. |

**Tabelle 5-13: Formulierungsbeispiel 13, Pearlized Shampoo**

| | |
|---|---|
| TEXAPON® NSO, Cognis, 28%-ig (INCI: Sodium Laureth Sulfate) | 32,00 |
| Cap01 | 0,50 |
| Perfume | 0,25 |
| Water | 55,25 |
| TEGO® Betain F 50, Evonik Goldschmidt GmbH, 38%-ig (INCI: Cocamidopropyl Betaine) | 8,00 |
| TEGO® Pearl N 300 Evonik Goldschmidt GmbH (INCI: Glycol Distearate; Laureth-4; Cocamidopropyl Betaine) | 2,00 |
| ANTIL® 171 Evonik Goldschmidt GmbH (INCI: PEG-18 Glyceryl Oleate/Cocoate) | 1,50 |
| NaCl | 0,50 |
| Preservative | q.s. |

**Tabelle 5-14: Formulierungsbeispiel 14, Rinse-Off Conditioner**

| | |
|---|---|
| Water | 90,20 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| Cap01 | 0,80 |
| TEGO® Alkanol 1618, Evonik Goldschmidt GmbH (INCI: Cetearyl Alcohol) | 5,00 |
| Preservative, Perfume | q.s. |

**Tabelle 5-15: Formulierungsbeispiel 15, Clear Conditioning Shampoo**

| | |
|---|---|
| TEXAPON® NSO, Cognis, 28%-ig (INCI: Sodium Laureth Sulfate) | 32,00 |
| ANTIL® 200, Evonik Goldschmidt GmbH (INCI: PEG-200 Hydrogenated Glyceryl Palmate; PEG-7 Glyceryl Cocoate) | 2,00 |
| Cap01 | 1,00 |
| Perfume | 0,25 |
| Water | 56,25 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00 |
| NaCl | 0,30 |
| Preservative | q.s. |

**Tabelle 5-16: Formulierungsbeispiel 16, feuchtigkeitspendendes Hautreinigungsmittel**

| | | |
|---|---|---|
| A | TEXAPON® NSO, Cognis, 28%-ig, (INCI: Sodium Laureth Sulfate) | 30,00 |
| | Cap01 | 0,70 |
| | Perfume | 0,30 |
| B | Water | 56,10 |
| | TEGOCEL® fluid HPM 4000, Evonik Goldschmidt GmbH, (INCI: Hydroxypropyl Methylcellulose) | 1,20 |
| | TEGO® Betain C 60, Evonik Goldschmidt GmbH, 46%-ig, (INCI: Cocamidopropyl Betaine) | 8,10 |
| | TEGOSOFT® APM, Evonik Goldschmidt GmbH, (INCI: PPG-3 Myristyl Ether) | 1,00 |
| | Cutina TS, Cognis (INCI:PEG- 3 Distearate) | 1,00 |
| | REWODERM® LI S 80, Evonik Goldschmidt GmbH, (INCI: PEG-200 Hydrogenated Glyceryl Palmate; PEG-7 Glyceryl Cocoate) | 1,00 |
| | Preservative | 0,60 |
| | Citric Acid, 30%-ig | q.s. |

**Tabelle 5-17: Formulierungsbeispiel 17, Duschgel**

| | |
|---|---|
| TEXAPON® NSO, Cognis, 28%-ig (INCI: Sodium Laureth Sulfate) | 15,00 |
| Cap01 | 0,50 |
| Perfume | 0,30 |
| PGFAC-S, Cognis (INCI: Sodium cocoyl hydrolyzed wheat protein glutamate) | 1,50 |
| REWOPOL SB CS 50 B, Evonik Goldschmidt GmbH, 40%-ig, (INCI: Disodium PEG-5 Laurylcitrate Sulfosuccinate; Sodium Laureth Sulfate) | 7,50 |
| Water | 59,60 |
| TEGO® Betain F 50, Evonik Goldschmidt GmbH, 38%-ig, (INCI: Cocamidopropyl Betaine) | 9,00 |
| TEGO® Betain 810, Evonik Goldschmidt GmbH, 38%-ig, (INCI: Capryl/Capramidopropyl Betaine) | 4,00 |
| Polyquaternium-7, Nalco, (INCI: Merquat 550) | 0,50 |
| ANTIL® 200, Evonik Goldschmidt GmbH, (INCI: PEG-200 Hydrogenated Glyceryl Palmate; PEG-7 Glyceryl Cocoate) | 1,80 |
| Preservative | 0,30 |

**Tabelle 5-18: Formulierungsbeispiel 18, Körperreinigungsmittel**

| | | |
|---|---|---|
| A | TEXAPON® NSO Cognis 28%-ig,(INCI: Sodium Laureth Sulfate | 30,00 |
| | Cap01 | 0,50 |
| | ABIL® B 8832, Evonik Goldschmidt GmbH, (INCI: Bis-PEG/PPG-20/20 Dimethicone) | 0,30 |
| | Perfume | 0,30 |
| B | Water | 53,00 |
| | TEGOCEL® fluid HPM 4000, Evonik Goldschmidt GmbH, (INCI: Hydroxypropyl Methylcellulose) | 1,20 |
| | Citric Acid Monohydrate | 0,50 |
| | REWOTERIC® AM C, Evonik Goldschmidt GmbH, 32%-ig, (INCI: Sodium Cocoamphoacetate) | 10,00 |
| | Cutina TS, Cognis (INCI:PEG- 3 Distearate) | 2,00 |
| | REWODERM® LI S 80, Evonik Goldschmidt GmbH, (INCI: PEG-200 Hydrogenated Glyceryl Palmate; PEG-7 Glyceryl Cocoate) | 1,60 |
| | Preservative | 0,60 |
| | Citric Acid, 30%-ig | q.s. |

**Tabelle 5-19: Formulierungsbeispiel 19, Body cleansing Foam**

| | |
|---|---|
| TEXAPON® NSO, Cognis, 28%-ig (INCI: Sodium Laureth Sulfate) | 14 |
| Perfume | 0,3 |
| Cap01 | 0,2 |
| REWOTERIC® AM C, Evonik Goldschmidt GmbH, 32%-ig (INCI: Sodium Cocoamphoacetate) | 8 |
| Water | 75,3 |
| TEGOCEL® HPM 50, Evonik Goldschmidt GmbH (INCI: Hydroxypropyl Methylcellulose) | 0,5 |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1 |
| Panthenol, BASF, (INCI: D-Panthenol USP) | 0,2 |
| Citric Acid Monohydrate | 0,5 |

**Tabelle 5-20: Formulierungsbeispiel 20, Rinse-Off Conditioner**

| | |
|---|---|
| Water | 90,20 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| Cap01 | 0,80 |
| TEGO® Alkanol 16, Evonik Goldschmidt GmbH (INCI:Cetyl Alkohol) | 5,00 |
| Preservative, Perfume | q.s. |

**Tabelle 5-21: Formulierungsbeispiel 21, Rinse-Off Conditioner**

| | |
|---|---|
| Water | 90,20 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| Cap01 | 0,80 |
| TEGO® Alkanol 18, Evonik Goldschmidt GmbH, (INCI: Stearyl Alkohol) | 5,00 |
| Preservative, Perfume | q.s. |

**Tabelle 5-22: Formulierungsbeispiel 22, Rinse-Off Conditioner**

| | |
|---|---|
| Water | 89,20 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| Cap01 | 0,80 |
| TEGO® Alkanol 1618, Evonik Goldschmidt GmbH (INCI: Cetearyl Alcohol) | 5,00 |
| DC 949, Dow Corning, (INCI: Amodimethicone) | 1,00 |
| Preservative, Perfume | q.s. |

**Tabelle 5-23: Formulierungsbeispiel 23, Rinse-Off Conditioner**

| | |
|---|---|
| Water | 89,20 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| Cap01 | 0,80 |
| TEGO® Alkanol 1618, Evonik Goldschmidt GmbH (INCI: Cetearyl Alcohol) | 5,00 |
| DC 1503 Fluid, Dow Corning, (INCI: Dimethicone, Dimethiconol) | 1,00 |
| Preservative, Perfume | q.s. |

**Tabelle 5-24: Formulierungsbeispiel 24, Turbid Conditioning Shampoo**

| | |
|---|---|
| TEXAPON® NSO, Cognis, 28%-ig (INCI: Sodium Laureth Sulfate) | 32,00 |
| ANTIL® 200, Evonik Goldschmidt GmbH (INCI: PEG-200 Hydrogenated Glyceryl Palmate; PEG-7 Glyceryl Cocoate) | 2,00 |
| Cap01 | 1,00 |
| Perfume | 0,25 |
| Water | 53,25 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00 |
| DC1503 Fluid, Dow Corning, (INCI: Dimethicone, Dimethiconol) | 1,00 |
| TEGO® Pearl N 300 Evonik Goldschmidt GmbH (INCI: Glycol Distearate; Laureth-4; Cocamidopropyl Betaine) | 2,00 |
| NaCl | 0,30 |
| Preservative | q.s. |

**Tabelle 5-25: Formulierungsbeispiel 25, Conditioning Anti- Dandruff Shampoo**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Zinc-Pyrion NF, WeylChem, 48%ig (INCI: Zinc Pyrithione) | 2,00 |
| | ABIL® Quat 3272, Evonik Goldschmidt GmbH, (INCI: Quaternium- 80) | 1,00 |
| C | Water | 36,7 |
| | TEGO® Carbomer 341 ER, Evonik Goldschmidt GmbH, (INCI: Acrylates/ C10-30 Alkyl Acrylate Crosspolymer) | 0,20 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Cap01 | 3,70 |
| | Preservative | q.s |

**Tabelle 5-26: Formulierungsbeispiel 26, Conditioning Anti- Dandruff Shampoo**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Crinipan AD, Haarmann & Reimer Fragrance GmbH (INCI: Climbazol) | 0,30 |
| | ABIL® Quat 3272, Evonik Goldschmidt GmbH, (INCI: Quaternium- 80) | 1,00 |
| C | Water | 38,4 |
| | TEGO® Carbomer 341 ER, Evonik Goldschmidt GmbH, (INCI: Acrylates/ C10-30 Alkyl Acrylate Crosspolymer) | 0,20 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Cap01 | 3,70 |
| | Preservative | q.s |

**Tabelle 5-27: Formulierungsbeispiel 27, Conditioning Anti- Dandruff Shampoo**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Zinc-Pyrion NF, WeylChem, 48%ig (INCI: Zinc Pyrithione) | 2,00 |
| | ABIL® Quat 3272, Evonik Goldschmidt GmbH, (INCI: Quaternium- 80) | 1,00 |
| C | Water | 36,4 |
| | TEGO® Carbomer 140, Evonik Goldschmidt GmbH, (INCI: Carbomer) | 0,50 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Cap01 | 3,70 |
| | Preservative | q.s |

**Tabelle 5-28: Formulierungsbeispiel 28, Conditioning Anti- Dandruff Shampoo**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Piroctone Olamine, Clariant (INCI: Octoprirox) | 0,30 |
| | ABIL® Quat 3272, Evonik Goldschmidt GmbH, (INCI: Quaternium- 80) | 1,00 |
| C | Water | 38,4 |
| | TEGO® Carbomer 341 ER, Evonik Goldschmidt GmbH, (INCI: Acrylates/ C10-30 Alkyl Acrylate Crosspolymer) | 0,20 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Cap01 | 3,70 |
| | Preservative | q.s |

## Patentansprüche

1. Zusammensetzung bestehend aus einer Mischung, die mindestens ein Isostearinsäureamid, mindestens einen Glycerinester und Wasser enthält, **dadurch gekennzeichnet, dass** der Glycerinester als Säurekomponente solche Carbonsäuren aufweist, die 8 bis 12 Kohlenstoffatome aufweisen und dass der Anteil an Isostearinsäureamid an der Mischung von 50 bis 95 Gew.-% bezogen auf das Gesamtgewicht der Mischung beträgt.

2. Zusammensetzung nach Anspruch 1, **dadurch gekennzeichnet, dass** die Mischung als Isostearinsäureamid ein Amid enthält, welches durch Umsetzung von Isostearinsäure mit 1-Aminopropan-2-ol erhalten wird.

3. Zusammensetzung nach Anspruch 1 oder 2, **dadurch gekennzeichnet, dass** die Mischung als Glycerinester Glycerinlaurat, vorzugsweise Glycerinmonolaurat enthält.

4. Zusammensetzung nach einem der Ansprüche 1 bis 3, **dadurch gekennzeichnet, dass** der Anteil an Glycerinester an der Mischung von 1 bis 45 Gew.-% bezogen auf das Gesamtgewicht der Mischung beträgt.

5. Zusammensetzung nach einem der Ansprüche 1 bis 4, **dadurch gekennzeichnet, dass** der Anteil an Wasser an der Mischung von 1 bis 15 Gew.-% bezogen auf das Gesamtgewicht der Mischung beträgt.

6. Zusammensetzung nach einem der Ansprüche 1 bis 5, **dadurch gekennzeichnet, dass** die Zusammensetzung ein Verdickungsmittel ist und/oder als Verdickungsmittel verwendet wird.

7. Verwendung einer Zusammensetzung nach einem der Ansprüche 1 bis 6 zur Herstellung eines Shampoos, Konditionierers, Duschgels, Körperreinigungsmittels oder Hautreinigungsmittels.

8. Verfahren zur Herstellung von Shampoos, Konditionierern, Duschgelen, Körperreinigungsmitteln oder Hautreinigungsmitteln, **dadurch gekennzeichnet, dass** eine Zusammensetzung gemäß einem der Ansprüche 1 bis 6 mit einer oder mehreren weiteren Komponenten gemischt wird.

9. Verfahren nach Anspruch 8, **dadurch gekennzeichnet, dass** eine Zusammensetzung gemäß einem der Ansprüche 1 bis 6 mit Wasser und einer oder mehreren weiteren Komponenten gemischt wird, wobei zumindest soviel Wasser zugemischt wird, dass der Anteil des Wassers an der Gesamtzusammensetzung größer 35 Gew.-% beträgt.

## Claims

1. Composition consisting of a mixture which comprises at least one isostearamide, at least one glycerol ester and water, **characterized in that** the glycerol ester has, as acid component, those carboxylic acids which have 8 to 12 carbon atoms and **in that** the fraction of isostearamide in the mixture is from 50 to 95% by weight, based on the total weight of the mixture.

2. Composition according to Claim 1, **characterized in that** the mixture comprises, as isostearamide, an amide which is obtained by reacting isostearic acid with 1-aminopropan-2-ol.

3. Composition according to Claim 1 or 2, **characterized in that** the mixture comprises, as glycerol ester, glycerol laurate, preferably glycerol monolaurate.

4. Composition according to one of Claims 1 to 3, **characterized in that** the fraction of glycerol ester in the mixture is from 1 to 45% by weight, based on the total weight of the mixture.

5. Composition according to one of Claims 1 to 4, **characterized in that** the fraction of water in the mixture is from 1 to 15% by weight, based on the total weight of the mixture.

6. Composition according to one of Claims 1 to 5, **characterized in that** the composition is a thickener and/or is used as a thickener.

7. Use of a composition according to one of Claims 1 to 6 for producing a shampoo, conditioner, shower gel, body cleaning composition or skin cleaning composition.

8. Process for producing shampoos, conditioners, shower gels, body cleaning compositions or skin cleaning compositions, **characterized in that** a composition according to one of Claims 1 to 6 is mixed with one or more further components.

9. Process according to Claim 8, **characterized in that** a composition according to one of Claims 1 to 6 is mixed with water and one or more further components, where at least sufficient water is mixed in for the fraction of the water in the overall composition to be greater than 35% by weight.

## Revendications

1. Composition constituée par un mélange, qui contient au moins un amide de l'acide isostéarique, au moins un ester de glycérine et de l'eau, **caractérisée en ce que** l'ester de glycérine comprend, en tant que composant acide, des acides carboxyliques qui comprennent 8 à 12 atomes de carbone, et **en ce que** la proportion d'amide de l'acide isostéarique dans le mélange est de 50 à 95 % en poids, par rapport au poids total du mélange.

2. Composition selon la revendication 1, **caractérisée en ce que** le mélange contient en tant qu'amide de l'acide isostéarique un amide qui est obtenu par mise en réaction d'acide isostéarique avec du 1-aminopropan-2-ol.

3. Composition selon la revendication 1 ou 2, **caractérisée en ce que** le mélange contient en tant qu'ester de glycérine du laurate de glycérine, de préférence du monolaurate de glycérine.

4. Composition selon l'une quelconque des revendications 1 à 3, **caractérisée en ce que** la proportion d'ester de glycérine dans le mélange est de 1 à 45 % en poids, par rapport au poids total du mélange.

5. Composition selon l'une quelconque des revendications 1 à 4, **caractérisée en ce que** la proportion d'eau dans le mélange est de 1 à 15 % en poids, par rapport au poids total du mélange.

6. Composition selon l'une quelconque des revendications 1 à 5, **caractérisée en ce que** la composition est un épaississant et/ou est utilisée en tant qu'épaississant.

7. Utilisation d'une composition selon l'une quelconque des revendications 1 à 6 pour la fabrication d'un shampoing, d'un après-shampoing, d'un gel douche, d'un produit de nettoyage pour le corps ou d'un produit de nettoyage pour la peau.

8. Procédé de fabrication de shampoings, d'après-shampoings, de gels douche, de produits de nettoyage pour le corps ou de produits de nettoyage pour la peau, **caractérisé en ce qu'**une composition selon l'une quelconque des revendications 1 à 6 est mélangée avec un ou plusieurs composants supplémentaires.

9. Procédé selon la revendication 8, **caractérisé en ce qu'**une composition selon l'une quelconque des revendications 1 à 6 est mélangée avec de l'eau et un ou plusieurs composants supplémentaires, au moins suffisamment d'eau pour que la proportion d'eau dans la composition totale soit supérieure à 35 % en poids étant incorporée.
